(19) Europäisches Patentamt
European Patent Office
Office européen des brevets

(11) Publication number: **0 386 972 B1**

(12) **EUROPEAN PATENT SPECIFICATION**

(45) Date of publication of patent specification: **04.08.93 Bulletin 93/31**

(51) Int. Cl.[5]: **C07D 519/04**

(21) Application number: **90302298.6**

(22) Date of filing: **05.03.90**

# (54) Process for the preparation of binary indole alkaloids.

(30) Priority: **04.03.89 JP 50988/89
04.03.89 JP 50989/89
04.03.89 JP 50990/89
04.03.89 JP 50991/89
08.09.89 JP 231659/89
02.11.89 JP 284966/89
28.07.89 JP 194306/89**

(43) Date of publication of application: **12.09.90 Bulletin 90/37**

(45) Publication of the grant of the patent: **04.08.93 Bulletin 93/31**

(84) Designated Contracting States: **AT BE CH DE DK ES FR GB GR IT LI LU NL SE**

(56) References cited:
**DE-A- 3 801 450
GB-A- 1 551 054
COMPTES RENDUS DE L'ACADEMIE DES SCIENCES, serie C, 22nd January 1979, pages 129-132, Paris, FR; P. MANGANEY et al.: "Hémisynthèse de la vinblastine, de la vincristine et de la leurosidine, alcaloides antitumoraux isolés de Catharanthus roseus G. Don (Apocynacées)"**

(73) Proprietor: **MITSUI PETROCHEMICAL INDUSTRIES, LTD.
2-5, Kasumigaseki 3-chome Chiyoda-ku
Tokyo 100 (JP)**

(72) Inventor: **Sakamoto, Naoya, c/o Mitsui Petrochem. Ind., Ltd.,
1-2, Waki 6-chome, Waki-cho
Kuga-gun, Yamaguchi-ken (JP)**
Inventor: **Tan, Hiroaki, c/o Mitsui Petrochem. Ind., Ltd.,
1-2, Waki 6-chome, Waki-cho
Kuga-gun, Yamaguchi-ken (JP)**
Inventor: **Hata, Eiichirou, c/o Mitsui Petrochem. Ind., Ltd.
1-2, Waki 6-chome, Waki-cho
Kuga-gun, Yamaguchi-ken (JP)**
Inventor: **Kihara, Noriaki, c/o Mitsui Petrochem. Ind., Ltd.
1-2, Waki 6-chome, Waki-cho
Kuga-gun, Yamaguchi-ken (JP)**

(74) Representative: **Myerscough, Philip Boyd et al
J.A.Kemp & Co. 14, South Square Gray's Inn
London, WC1R 5EU (GB)**

**EP 0 386 972 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to a process for the preparation of binary indole alkaloids, such as vinblastine or leurosidine, which are effective as an anti-cancer drug.

Conventional processes for the preparations, e.g. through chemical transformation of anhydrovinblastine (AVLB), of binary indole alkaloids possessing an anti-tumor activity, such as vinblastine (VLB) and leurosidine (LEU) include e.g. the process disclosed in J.C.S. Chem. Commn. 583, 1979. However, the yields of object vinblastine (VLB) and leurosidine (LEU), etc., are very poor as low as 1 to 2%. Also, the processes employing an enzyme are disclosed in J.C.S. Chem. Commn. 257, 1979 and Phytochemistry 26(12), 3233 (1987). In these processes, however, there are disadvantages, such as very low yields, long reaction times, etc. Hence, both of the processes had problems as a process for industrial production and thus were not satisfactory processes.

### SUMMARY OF THE INVENTION

The problem to be solved by the present invention resides in dissolving the problems of the above conventional technologies, and the object of this invention is to provide an industrially advantageous process for the preparation, where binary indole alkaloids, which are effective as an anti-cancer drug, such as vinblastine, leurosidine, or the like, can be prepared in a higher yield.

For accomplishment of the above object, the present inventors employed e.g. anhydrovinblastine (AVLB) as a starting material and attempted several types of hydration reactions, redox reactions, etc. As a result, they have found out that vinblastine (VLB), leurosidine (LEU), etc., which are effective as an anti-cancer drug, can be synthesized in one step in a higher yield, from anhydrovinblastine (AVLB), by addition of a hydride source to the solution containing AVLB in the presence of trivalent iron and oxygen, whereby they have arrived at the completion of the present invention.

That is, the present invention relates to a process for the preparation of a compound represented by the general formula (II):

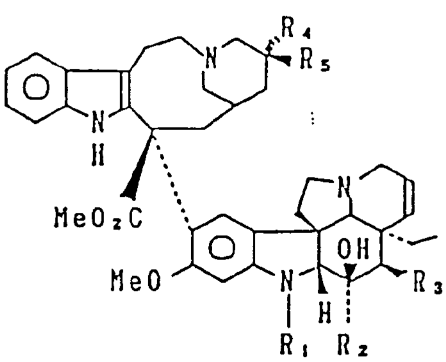

( II )

(wherein $R_1$ stands for a hydrogen atom, a lower alkyl group or formyl group, $R_2$ stands for a lower alkoxycarbonyl group or amide group, $R_3$ stands for an acetoxy group or hydroxy group $R_4$ stands for an OH group, $R_5$ stands for an ethyl group or $R_4$ stands for an ethyl group, $R_5$ stands for an OH group) by employment, as a starting material, of a compound or a salt thereof represented by the general formula (I ):

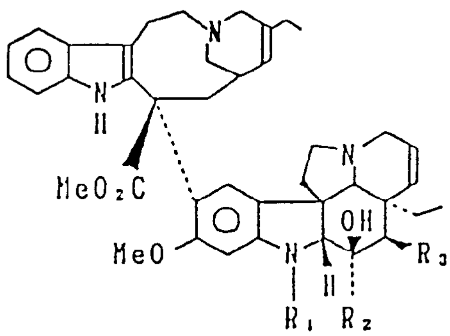

( I )

(wherein $R_1$ to $R_3$ are the same defined in the formula (II)), characterized in that a trivalent iron source and a hydride source are added in the presence of oxygen.

A representative of the process for the preparation according to the present invention can be exemplified as a process wherein vinblastine (referred to as VLB, hereinafter) represented, as shown below, by the formula (V) (in the formula (V), $R_1$ stands for an OH group, and $R_2$ stands for an ethyl group) and leurosidine (referred to as LEU, hereinafter) (in the formula (V), $R_1$ stands for an ethyl group, and $R_2$ stands for an OH group) are synthesized from anhydrovinblastine (referred to as AVLB, hereinafter) represented by the formula (IV), as a starting material. As other object compounds of the present invention, a compound of the above formula (II) wherein $R_1$ is a hydrogen atom can include e.g. N-desmethylvinblastine, etc., and a compound of the formula (II) wherein $R_1$ is a formyl group can include e.g. vincristine, etc., those of which can be synthesized from its corresponding compound of formula (I) as a starting material.

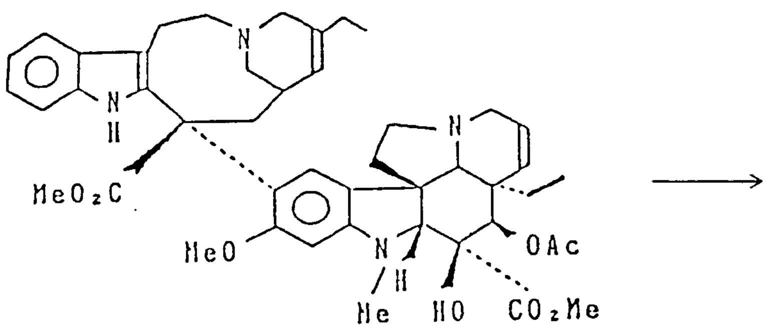

( IV )

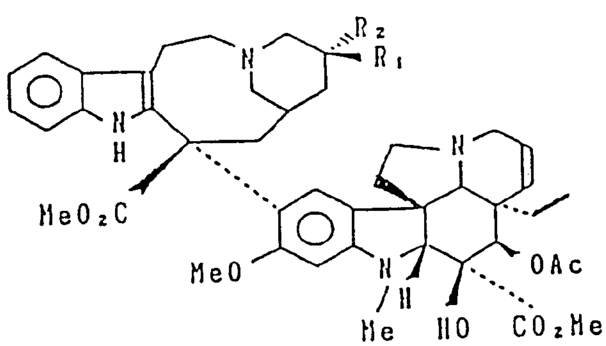

( V )

DETAILED DESCRIPTION OF THE INVENTION

The present invention can be carried out in a very simple process in which a hydride source is added to a suitable solvent dissolving a trivalent iron source and a compound or a salt thereof represented by the general formula (I), in the presence of oxygen.

The trivalent iron to be employed may be any if it can be dissolved in the reaction mixture and participate in the reaction. In particular, chlorides, sulfates, and nitrates are preferable. The amount of these to be added is 0.01 to 10,000-fold molar excess, preferably 1 to 2,000-fold molar excess, based on the starting material.

Solvents to be employed in this process can be exemplified as $H_2O$, alcohols, such as methanol, ethanol, etc., THF, acetonitrile, DMF, DMSO, etc. A mixture solvent of 2 or more of these may also be employed. Preferably employed is $H_2O$ singly, or a mixture solvent of $H_2O$-methanol.

Hydride sources to be added can include sodium borohydride, potassium borohydride, sodium borocyanohydride, and amine complexes of borane. The hydride source is added in an amount of 0.05 to 10-fold molar excess, preferably 0.1 to 5-fold molar excess, based on the trivalent iron.

An oxygen source may be oxygen or inert gas-diluted oxygen, but normally, air is preferred. For one of preferable processes, an oxygen-containing gas is bubbled through a reaction solution for a determined time so as to dissolve an suitable amount of oxygen in the reaction solvent, prior to the addition of the hydride source.

As the reaction temperature, a wide range from a melting point of reaction solvent to approx. 50°C, preferably -20 to 10°C, can be adopted.

In the process for the preparation according to the present invention, further addition of (i) an oxalic acid ion source and/or a malonic acid ion source, (ii) an inorganic anion source, or (iii) an amino acid can further raise the yield of an object compound. Also, in the case of the above described (i), (a) it is further preferred to add a dicarboxylic acid or a salt thereof represented by the general formula (III), and further addition of amino acid thereto is more preferable, or

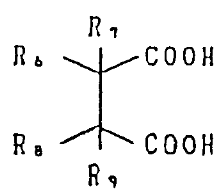

(III)

(wherein $R_7$ and $R_9$ stand for a hydrogen atom or a lower alkyl group, $R_6$ and $R_6$ stand for a hydrogen atom or a lower alkyl group, or together form a double bond). (b) it is preferred to add pyridine or its derivative, and further addition of amino acid thereto is more preferable, or (c) it is preferred to add inorganic anion, and further addition of amino acid thereto is more preferable, or (d) addition of amino acid is preferable. In the case of the above described (ii) further addition of amino acid is preferable.

The sources of oxalic acid ion and malonic acid ion can be any of ones if it can be dissolved in the reaction solvent, to form a complex with the trivalent iron. In the cases of the addition of free oxalic acid and malonic acid, however, it is necessary to add further a suitable base in an equivalent amount to those of free acid. Preferable examples of oxalic acid ion sources for the addition can include, as the type of a salt, alkali metal salts, such as an Li salt, Na salt, K salt, etc., an ammonium salt, and an alkyl ammonium salt. These are added in an amount of 0.1 to 10-fold molar excess, preferably 1 to 4-fold molar excess, based on the trivalent iron. Also, the addition of a previously prepared complex salt of trivalent iron-oxalic acid and/or a complex salt of trivalent iron-malonic acid is one of the preferable processes, as the simultaneous addition of an oxalic acid source and/or a malonic acid source and a trivalent iron source. The complex salts of trivalent iron-oxalic acid can be exemplified as e.g. $(NH_4)_3Fe(C_2O_4)_3$, $K_3Fe(C_2O_4)_3$, etc., and the complex salts of malonic acid salts can include e.g. $(NH_4)_3Fe(C_3O_4)_3$, $K_3Fe(C_3H_2O_4)_3$, etc.

The inorganic anion sources are not particularly restricted. However, inorganic anions like $CO_3^{2-}$ and $PO_4^{3-}$ - which form insoluble iron salts - are excluded. In the present invention, particularly preferred are $Cl^-$, $Br^-$, $I^-$, $SO_4^{2-}$, etc. The type of a salt of an inorganic anion source can be any of ones if they can be dissolved in the reaction solvent. In particular, an ammonium salt, an alkylammonium salt, and an alkali metal salt are preferred. An addition amount of these is 0.1 to 100-fold molar excess, preferably 1 to 10-fold molar excess, based on the trivalent iron.

The amino acid can be any of amino acids, but preferable are the ones of a lower molecular weight, such as glycine, etc. More preferable are glycine and N-methylglycine. Its addition amount is 0.01 to 1,000-fold molar excess, preferably 1 to 10-mold molar excess, based on the trivalent iron.

The compounds represented by the formula (III) include maleic acid, citraconic acid, succinic acid, itaconic acid, etc., however, they are not restricted to these exemplified compounds. In particular, maleic acid and succinic acid can be cited as preferable examples.

It is preferred that these compounds are added especially in the form of salts. The type of a salt may be any, though particularly preferred are an ammonium salt and an alkyl ammonium salt. In the case where a

free salt is added, it is required to further add a suitable salt base in an equivalent amount to free acids or so. They are added in amount of 0.1 to 3-fold molar excess, preferably 0.3 to 2-fold molar excess, based on the trivalent iron.

Pyridine or its derivatives include pyridine derivatives, such as pyridine, alkylpyridine, etc., α,α'-bipyridyl derivatives, such as α,α'-bipyridyl, alkyl α,α'-bipyridyl, etc. In particular, α,α'-bipyridyl is preferable. Its addition amount is 0.01 to 10-fold molar excess, preferably 0.1 to 3-fold molar excess, based on the trivalent iron.

The process for the preparation according to the present invention can be made continuous by allowing the respective compounds of (I) type compound, a hydride source, oxygen, and a trivalent iron to be included in two or more solutions, and then by allowing said two or more solutions to continuously contact with each other, or more preferably by allowing an oxygen- and trivalent iron- and (I) type compound-containing solution to continuously contact with a hydride source-contacting solution.

This continuous process for the preparation can be carried out in a very simple operation in which e.g. the above-described two or more solutions are simultaneously and continuously fed into the inlet of one end of a reaction column, to be allowed to contact and mix with each other, whereby the reaction is completed while the solutions are past through the column, so that the reaction mixture is continuously recovered from the outlet of another side of the column.

The yield of an object compound can be significantly increased by addition of one or more components selected from a pyridine derivative, an inorganic anion source, a dicarboxylic acid or salt thereof represented by the formula (III), and an amino acid, together with an oxalic acid source and/or malonic acid source, to any solution to be contacted and mixed in this continuous process.

Usually, these addition components are most preferably added to a solution which contains a trivalent iron source. To this, however, the addition method is not restricted. The yield is increased, almost similarly as above, by addition of the components to any of two or more solutions to be contacted.

However, the addition of a combination by which the hydride source is rapidly degraded should be avoided.

The pyridine derivatives may be any if those are a pyridine derivatives. Particularly preferred are pyridine, alkylpyridines, α,α'-bipyridine, alkyl α,α'-bipyridines. Its addition amount is 0.01 to 10-fold molar excess based on the trivalent iron.

It is preferable than an oxygen source is suitably dissolved in any of the solutions to be contacted, by previously bubbling an oxygen-containing gas.

Contact time for the reaction solutions is 0.1 sec. to 60 min., preferably 1 sec. to 10 min.

The reaction vessel may be any shapes; a column-shaped reaction vessel is particularly preferred.

As compared with conventional processes, the process according to the present invention has the advantages that vinblastine (VLB), leurosidine (LEU), etc. - which are effective as an anti-cancer drug - can be obtained e.g. from anhydrovinblastine (AVLB), in a higher yield and easier operation.

Taking into consideration that vinblastine (VLB) is a very high-priced anti-cancer drug, the effects brought by the present invention are extremely high.

[Examples]

The present invention is more specifically illustrated below. By this examples, however, the present invention is not restricted.

Example 1

To a 50 ml-reaction vessel were put 10 ml of $H_2O$, 1.9 mg of AVLB, 1 ml of an aqueous solution of $FeCl_36H_2O$ (1.2 M), and 0.024 ml of an aqueous solution of 2 N HCl, and the mixture was stirred for 30 min. under cooling with ice while air was bubbled. Following addition of 1 ml of an aqueous solution of $NaBH_4$ (0.227 M), the mixture was stirred for an additional 30 min. Then, 2 ml of 25% ammonia water was added, whereby the solution was made basic. The products were extracted by addition of 10 ml of ethyl acetate, 3 times. The extracts were combined and evaporated to dryness under reduced pressure at 40°C or less. The resulting sample was analyzed by high-performance liquid chromatography (HPLC) under the conditions described below. At this time, the conversion rate of AVLB was 95%, the yield of VLB was 10%, and the yield of LEU was 6%.

(Analytical Conditions)

| | |
|---|---|
| Column: | YMC-packed column A-512(CN) 6 x 150 mm |
| Mobile phase: | $H_2O$:MeOH:$Et_3N$:AcOH = 1500:1500:4:1.6 (V/V/V/V) |
| Flow rate: | 1 ml/min |

Column temperature: 45°
Detection: UV (254 nm)
Retention time: VLB 19.5 min
LEU 17 min
AVLB 46 min

Examples 2 to 4

To procedure was carried out in the same manner as in Example 1 except that glycine was further added in a predetermined amount. The results are shown in Table 1.

Table 1

| Example | Glycine/$Fe^{3+}$ (Molar ratio) | Conversion rate of AVLB (%) | Yield of VLB (%) | Yield of LEU (%) |
|---|---|---|---|---|
| 2 | 2 | 90 | 15 | 10 |
| 3 | 4 | 87 | 21 | 12 |
| 4 | 5 | 85 | 14 | 9 |

Example 5

The procedure was carried out in the same manner as in Example 3 except that glycine in Example 3 was replaced by N-methylglycine. At this time, the conversion rate of AVLB was 66%, the yield of VLB was 24%, and the yield of LEU was 14%.

Examples 6 to 8

The procedure was carried out in the same manner as in Example 3 except that the amounts of $Fe^{3+}$ and $NaBH_4$ in Example 3 were varied. The results are shown in Table 2.

Table 2

| Example | Addition amount of $Fe^{3+}$aq (cc) | Addition amount of $NaBH_4$ aq (cc) | Conversion rate of AVLB (%) | Yield of VLB (%) | Yield of LEU (%) |
|---|---|---|---|---|---|
| 6 | 0.4 | 0.4 | 32 | 7 | 3 |
| 7 | 0.5 | 0.5 | 35 | 9 | 4 |
| 8 | 2 | 2 | 69 | 16.5 | 9 |

Examples 9 to 11

The procedure was carried out in the same manner as in Example 3 except that the amount of $NaBH_4$ in Example 3 was varied. The results are shown in Table 3.

Table 3

| Example | Addition amount of $NaBH_4$ aq (cc) | Conversion rate of AVLB (%) | Yield of VLB (%) | Yield of LEU (%) |
|---|---|---|---|---|
| 9 | 0.5 | 29 | 10.5 | 6 |
| 10 | 2 | 69 | 11.5 | 7 |
| 11 | 5 | 91 | 16 | 9 |

Examples 12 to 14

The procedure was carried out in the same manner as in Example 3 except that the $FeCl_3 6H_2O$ solution in Example 3 was replaced by other iron salt solutions. The results are shown in Table 4.

Table 4

| Example | Iron salt | Conversion rate of AVLB (%) | Yield of VLB (%) | Yield of LEU (%) |
|---|---|---|---|---|
| 12 | $Fe(NO_3)_3 \cdot 9H_2O$ | 20 | 4 | 2 |
| 13 | $Fe_2(SO_4)_3$ | 75 | 18 | 11 |
| 14 | $FeBr_3$ | 26.5 | 4 | 1 |

Examples 15 to 16

The procedure was carried out in the same manner as in Example 3 except that $NaBH_4$ in Example 3 was replaced by other hydride sources. The results are shown in Table 5.

Table 5

| Example | Hydride source | Conversion rate of AVLB (%) | Yield of VLB (%) | Yield of LEU (%) |
|---|---|---|---|---|
| 15 | $NaBH_3CN$ | 80 | 14 | 10 |
| 16 | $KBH_4$ | 89 | 19 | 11 |

Example 17

In a 50 ml-reaction vessel were placed 10 ml of $H_2O$, 1.9 mg of anhydrovinblastine, 500-fold molar excess $(NH_4)_3Fe(C_2O_4)_33H_2O$ based on the anhydrovinblastine, and 0.024 ml of an aqueous solution of 2 N HCl. The mixture was stirred under cooling with ice over 30 min., while air was bubbled. Then, 1 ml of an aqueous solution of $NaBH_4$ (0.227 M) was added, and the mixture was stirred for an additional 30 min. The mixture was made basic by addition of 2 ml of 25% ammonia water and was extracted with 10 ml of ethyl acetate, 3 times. The extracts were combined and evaporated to dryness under reduced pressure at 40°C or less. The products were analyzed by HPLC under the following conditions. At this time, the conversion rate of anhydrovinblastine (AVLB) was 97%, the yield of vinblastine (VLB) was 23%, and the yield of leurosidine (LEU) was 12%.

(Analytical Conditions)

| | |
|---|---|
| Column: | YMC-packed column AM-312 |
| Mobil phase: | Aqueous solution of 0.01 M ammonium carbonate : acetonitrile = 1800 : 1200 (V/V) |
| Flow rate: | 1 ml/min |
| Temperature: | 45°C |
| Detection wavelength: | UV (245 nm) |
| Retention time: | VLB 12.3 min<br>LEU 9.0 min<br>AVLB 39.1 min |

Example 18

The procedure was carried out in the same manner as in Example 17 except that 4-fold molar excess glycine based on the iron atom in Example 17 was added. At this time, the conversion rate of AVLB was 86%, the yield of VLB was 27%, and the yield of LEU was 14%.

Example 19

The procedure was carried out in the same manner as in Example 18 except that 1 ml of an aqueous solution of $FeCl_36H_2O$ (1.2 M) was added in place of $(NH_4)_3Fe(C_2O_4)_33H_2O$ in Example 18 and that 3-fold molar excess $(NH_4)_2C_2O_4$ based on the trivalent iron was further added. At this time, the conversion rate of AVLB was 86.5%, the yield of VLB was 34%, and the yield of LEU was 22.5%.

Examples 20 to 21

The procedure was carried out in the same manner as in Example 19 except that the addition amount of $(NH_4)_2C_2O_4$ in Example 19 was varied. The results are shown in Table 6.

Table 6

| Example | $C_2O_4^{2-}/Fe^{3+}$ (Molar ratio) | Conversion rate of AVLB (%) | Yield of VLB (%) | Yield of LEU (%) |
|---|---|---|---|---|
| 20 | 1 | 43 | 22.5 | 15 |
| 21 | 2 | 63 | 31.5 | 21.5 |

Examples 22 to 24

The procedure was carried out in the same manner as in Example 19 except that the amount of iron added was reduced to one-half and that the amount of $(NH_4)_2C_2O_4$ added was varied. The results are shown in Table 7.

Table 7

| Example | $C_2O_4^{2-}/Fe^{3+}$ (Molar ratio) | Conversion rate of AVLB (%) | Yield of VLB (%) | Yield of LEU (%) |
|---|---|---|---|---|
| 22 | 1 | 43.5 | 19.5 | 17.5 |
| 23 | 2 | 56 | 30.5 | 23.5 |
| 24 | 3 | 87.5 | 27.5 | 16.5 |

Example 25

The procedure was carried out in the same manner as in Example 19 except that the aqueous solution of $FeCl_36H_2O$ in Example 19 was replaced by an aqueous solution of $Fe(NO_3)_39H_2O$. The conversion rate of AVLB was 89.5%, the yield of VLB was 26%, and the yield of LEU was 15%.

Examples 26 to 28

The procedure was carried out in the same manner as in Example 19 except that the solvent was changed from $H_2O$ to other mixture solvents and that the reaction temperature was varied. The results are shown in Table 8.

Table 8

| Example | Solvent* | Reaction temperature (°C) | Conversion rate of AVLB (%) | Yield of VLB (%) | Yield of LEU (%) |
|---|---|---|---|---|---|
| 26 | $H_2O$-MeOH | -15°C | 74.5 | 36 | 23.5 |
| 27 | $H_2O$-DMSO | -8°C | 74 | 21 | 14 |
| 28 | $H_2O$-$CH_3CN$ | -6°C | 71 | 29.5 | 16 |

*$H_2O$: organic solvent = 8:2 (V/V)

Examples 29 to 33

The procedure was carried out in the same manner as in Example 19 except that $(NH_4)_2C_2O_4$ in Example 19 was replaced by other salts. The results are shown in Table 9.

Table 9

| Example | Added oxalic acid salt | Conversion rate of AVLB (%) | Yield of VLB (%) | Yield of LEU (%) |
|---|---|---|---|---|
| 29 | $H_2C_2O_4$ | 78 | 30 | 25.5 |
| 30 | $K_2C_2O_4$ | 78 | 30.5 | 22 |
| 31 | $(Me_4N^+)_2C_2O_4$ | 89.5 | 32 | 21 |
| 32 | $(Et_4N^+)_2C_2O_4$ | 55 | 21.5 | 7.5 |
| 33 | $(CH_3N^+H_3)_2C_2O_4$ | 82 | 36 | 26 |

Examples 34 to 35

The procedure was carried out in the same manner as in Example 19 except that $(NH_4)_2C_2O_4$ in Example 19 was replaced by ammonium malonate. The results are shown in Table 10.

Table 10

| Example | Conversion rate of AVLB (%) | Yield of VLB (%) | Yield of LEU (%) |
|---|---|---|---|
| 34 | 65 | 22 | 11 |
| 35* | 80 | 30 | 12 |

* After pH was adjusted to 6.6 to 6.7, an aqueous solution of $NaBH_4$ was added.

Example 36

To a 100 ml-reaction vessel equipped with a turning blade were put 22.3 mg of anhydrovinblastine sulfate, 100 ml of $H_2O$, 0.2029 g of $FeCl_36H_2O$, 0.2134 g of $(NH_4)_2C_2O_4$, 0.0874 g of maleic acid, and 25% ammonia water (0.1025 ml) equivalent to the maleic acid. The mixture was stirred at 0°C over 30 min. in a high speed with the turning blade while air was bubbled (50 ml/min). Then, 1 ml of aqueous 0.5 M $NaBH_4$ solution was fed with a pump in 1 min. Following stirring for an additional 30 min., the solution was made basic by addition of 5 ml of 25% ammonia water. The product was extracted with 100 ml of ethyl acetate, 3 times.

The extracts were combined and dried by means of $Na_2SO_4$, followed by being evaporated to dryness under reduced pressure at 40°C or less. The residues were measured up with methanol and analyzed by high performance liquid chromatography. At this time, the conversion rate of anhydroblastine (AVLB) was 94.5%, the yield of vinblastine (VLB) was 50%, leurosidine (LEU) was 16%, and the vinblastine selectivity was 52.9%.

(Analytical conditions)

| | |
|---|---|
| Column: | YMC-packed column AM-312 6 x 150 mm |
| Mobile phase: | Aqueous solution of 0.01 M aqueous ammonium carbonate solution : acetonitrile = 12 : 1800 (V/V) |
| Flow rate: | 1 ml/min |
| Temperature: | 45°C |
| Detection: | UV (254 mm) |
| Retention time: | VLB 13.0 min<br>LEU 10.5 min<br>AVLB 42.5 min |

Comparative Example 1

The procedure was carried out in the same manner as in Example 36 except that neither maleic acid nor ammonia water was added. At this time, the conversion rate of AVLB was 93.5%, the yield of VLB was 40%, the yield of LEU was 15.5%, and the VLB selectively was 42.8%.

Comparative Examples 37 to 40

The procedure was carried out in the same manner as in Example 36 except that the addition amounts of maleic acid and ammonia water were varied. The results are shown in Table 11.

Table 11

| Example | Maleic acid/$Fe^{3+}$ (mol/mol) | Conversion rate of AVLB (%) | Yield of VLB (%) | Yield of LEU (%) | VLB selectivity (%) |
|---|---|---|---|---|---|
| 37 | 0.5 | 90 | 42.5 | 13 | 47.2 |
| 38 | 0.75 | 88.5 | 47.5 | 14 | 53.7 |
| 39 | 1.5 | 87.5 | 49 | 10.5 | 56 |
| 40 | 2 | 81 | 40.5 | 10 | 50 |
| Comparative Example 1 | 0 | 93.5 | 40 | 15.5 | 42.8 |

$NH_3$ water was added in an amount equivalent to the maleic acid.

Comparative Examples 41 to 43

The procedure was carried out in the same manner as in Example 36 except that maleic acid was replaced by succinic acid and the addition amount was varied. The results are shown in Table 12.

Table 12

| Example | Succinic acid/$Fe^{3+}$ (mol/mol) | Conversion rate of AVLB (%) | Yield of VLB (%) | Yield of LEU (%) | VLB selectivity (%) |
|---|---|---|---|---|---|
| 41 | 0.75 | 97 | 43.5 | 13.5 | 44.8 |
| 42 | 1 | 93.5 | 47 | 12 | 50.3 |
| 43 | 1.5 | 87 | 44 | 11 | 50.6 |

$NH_3$ water was added in an amount equivalent to the succinic acid.

Examples 44 to 46

The procedure was carried out in the same manner as in Example 36 except that maleic acid in Example 36 was replaced by other dicarboxylic acids. The results are shown in Table 13.

Table 13

| Example | Dicarboxylic acid | Conversion rate of AVLB (%) | Yield of VLB (%) | Yield of LEU (%) | VLB selectivity (%) |
|---|---|---|---|---|---|
| 44 | Itaconic acid | 77.5 | 34 | 9 | 47.7 |
| 45 | Citraconic acid | 88.5 | 40 | 13 | 45.2 |
| 46 | Ketosuccinic acid | 76.5 | 34 | 12 | 45.8 |

Example 47

To a 50 ml-reaction vessel were put to 10 ml of $H_2O$, 1.9 mg of anhydrovinblastine, 1 ml of an aqueous solution of $3FeCl_36H_2O$ (1.5 M), 0.024 ml of 2 N HCl, 4-fold molar excess glycine, 6-fold molar excess $NH_4Cl$, based on $Fe^{3+}$. Following stirring the mixture over 30 min. under cooling with ice while air was bubbled, 1 ml of an aqueous solution of $NaBH_4$ (0.227 M) was added and stirred for an additional 30 min. Then, 2 ml of 25% ammonia water was added, thus making the solution basic. The product was extracted with 10 ml of ethyl acetate, 3 times. The extracts were combined and evaporated to dryness under reduced pressure at not higher than 40°C. The obtained sample was analyzed by high-performance liquid chromatography. At this time, the conversion rate of anhydrovinblastine (AVLB) was 66.5%, the yield of vinblastine (VLB) was 30.5%, and the yield of leurocidin (LEU) was 14.5%.

(Analysis conditions)

| | |
|---|---|
| Column: | YMC-packed column A-512 (CN) 6 x 150 mm |
| Mobile phase: | $H_2O$:MeOH:$Et_3N$:AcOH = 1500:1500:4:1.6 (V/V/V/V) |
| Flow rate: | 1 ml/min. |
| Temperature: | 45°C |
| Detection wavelength: | UV (254 nm) |
| Retention time: | VLB 19.5 min. |
| | LEU 17 min. |
| | AVLB 46 min. |

Example 48

The procedure was carried out in the same manner as in Example 47 except that $NH_4Cl$ in Example 47 was replaced by $NH_4Br$ in Example 47. At this time, the conversion rate of AVLB was 59.5%, the yield of VLB was 22%, the yield of LEU was 12.5%.

Example 49

The procedure was carried out in the same manner as in Example 47 except that $NH_4Cl$ in Example 47 was replaced by 3-fold molar excess $(NH_4)_2SO_4$ based on $Fe^{3+}$. At this time, the conversion rate of AVLB was 75%, the yield of VLB was 28%, and the yield of LEU was 15.5%.

Examples 50 to 52

The procedure was carried out in the same manner as in Example 47 except that the addition amount of $NH_4Cl$ in Example 47 was varied. The results are shown in Table 14.

Table 14

| Example | $NH_4Cl/Fe^{3+}$ (Molar ratio) | Conversion rate of AVLB (%) | Yield of VLB (%) | Yield of LEU (%) |
|---|---|---|---|---|
| 50 | 2 | 63 | 26 | 17.5 |
| 51 | 4 | 71 | 31.5 | 19 |
| 52 | 8 | 81.5 | 29 | 22.5 |

Examples 53 to 55

The procedure was carried out in the same manner as in Example 47 except that $NH_4Cl$ in Example 47 was replaced by other salts. The results are shown in Table 15.

Table 15

| Example | Added salt | Conversion rate of AVLB (%) | Yield of VLB (%) | Yield of LEU (%) |
|---|---|---|---|---|
| 53 | $(CH_3)_4NCl$ | 69.5 | 30 | 19 |
| 54 | $(CH_3)_2NH_2Cl$ | 70 | 30.5 | 22 |
| 55 | NaCl | 67 | 23 | 12.5 |

Examples 56

In a 50 ml-reaction vessel were placed 10 ml of $H_2O$, 1.9 mg of anhydrovinblastine, 1 ml of an aqueous solution of $FeCl_36H_2O$ (1.2 M), 0.024 ml of an aqueous 2 N HCl, and 2-fold molar excess $(NH_4)_2C_2O_4$, based on $Fe^{3+}$. The mixture was stirred over 30 min. under cooling in ice while air was bubbled. After 1 ml of an aqueous solution of $NaBH_4$ (0.227 M) was added, the mixture was further stirred over 30 min. By addition of 2 ml of 25% ammonia water, the mixture solution was made basic and extracted with 10 ml of ethyl acetate, 3 times. The extracts were combined and evaporated to dryness under reduced pressure at 40°C or less. The resulting products were analyzed by high-performance liquid chromatography. At this time, the conversion rate of anhydrovinblastine (AVLB) was 66.0%, the yield of vinblastine of (VLB) 35.5%, and the yield of leurosidine (LEU) was 18%.

Analytical conditions)

| | |
|---|---|
| Column: | YMC-packed column A-512(CN) 6 x 150 mm |
| Mobile phase: | $H_2O$:MeOH:$Et_3N$:AcOH = 1500:1500:4:1.6 |
| Flow rate: | 1 ml/min. |
| Temperature: | 45°C |
| Detection: | UV (254 nm) |

Retention time: VLB 19.5 min.
LEU 17 min.
AVLB 46 min.

Examples 57 to 59

The procedure was carried out in the same manner as in Example 56 except that the addition amount of $NH_4Cl$ in Example 56 was varied. The results are shown in Table 16.

Table 16

| Example | $NH_4Cl/Fe^{3+}$ (Molar ratio) | Conversion rate of AVLB (%) | Yield of VLB (%) | Yield of LEU (%) |
|---|---|---|---|---|
| 57 | 2 | 63.5 | 32.5 | 17 |
| 58 | 6 | 69 | 33.5 | 21 |
| 59 | 8 | 77.5 | 35 | 21 |

Examples 60 to 61

The procedure was carried out in the same manner as in Example 56 except that $NH_4Cl$ in Example 56 was replaced by $(NH_4)_2SO_4$. The results are shown in Table 17.

Table 17

| Example | $(NH_4)_3SO_4/Fe^{3+}$ (Molar ratio) | Conversion rate of AVLB (%) | Yield of VLB (%) | Yield of LEU (%) |
|---|---|---|---|---|
| 60 | 2 | 70.5 | 31.5 | 18 |
| 61 | 3 | 66 | 30 | 16 |

Example 62

In a 100 cc-reaction vessel equipped with a turning blade were placed 19 mg of AVLB, 100 ml of $H_2O$, 0.2025 g of $FCl_36H_2O$, 0.2162 g of $(NH_4)_2C_2O_4$, 0.1604 g of $NH_4Cl$, 0.225 g of glycine, and 0.12 ml of 2 N HCl. The mixture was stirred at 0°C over 30 min. in a high speed with the turbine blade while air was bubbled. Then, 2 ml of an aqueous solution of $NaBH_4$ (0.227 M) was dropped over ca. 1 min. By addition of 10 ml of 25% ammonia water, the solution was made basic, followed by being extracted with 100 ml of ethyl acetate, 3 times. The following procedure was performed in the same manner as in Example 56. At this time, the conversion rate of AVLB was 80%, the yield of VLB was 41%, and the yield of LEU was 18%.

Examples 63 to 64

The procedure was carried out in the same manner as in Example 62 except that the amount of $FeCl_36H_2O$ in Example 62 was varied. (The $(NH_4)_2C_2O_4/Fe^{3+}$, $NH_4Cl/Fe^{3+}$, and glycine/$Fe^{+3}$ ratios were identical with those of Example 62.) The results are shown in Table 18.

Table 18

| Example | $Fe^{3+}$/AVLB (Molar ratio) | Conversion rate of AVLB (%) | Yield of VLB (%) | Yield of LEU (%) |
|---|---|---|---|---|
| 63 | 42 | 77 | 39 | 19.5 |
| 64 | 16 | 86 | 37 | 14.5 |

Example 65

The procedure was carried out in the same manner as in Example 56 except that glycine in Example 56 was not added. At this time, the conversion rate of AVLB was 64%, the yield of VLB was 20%, and the yield of LEU was 16%.

Example 66

Following dissolving in 100 cc of water 19 mg of anhydrovinblastine (AVLB), 32-fold molar excess $FeCl_36H_2O$ and 5-fold molar excess 2 N HCl based on AVLB, 2-fold molar excess $(NH_4)_2C_2O_4$, 4-fold $NH_4Cl$, and 4-fold molar excess glycine, based on $Fe^{3+}$, air was bubbled over 30 min. at 0°C. This solution and a solution, which was prepared by dissolving 12.9 mg of $NaBH_4$ in 2 cc of water and cooling to 0°C, were introduced into a reaction column and were allowed to contact with each other. After the reaction was proceeded by passing the solution through the column for 1 min., the reaction solution was introduced into ammonia water (prepared by dilution of 2 ml of 25% ammonia water with 5 ml of water).

This mixture was extracted with 100 ml of AcOEt, 3 times. The eluate was evaporated to dryness under reduced pressure at not higher than 40°C. The analysis of the product by high-performance liquid chromatography showed that the conversion rate of AVLB was 67.5%, the yield of vinblastine (VLB) was 32%, and the yield of leurosidine (LEU) was 12%.

The analytical conditions of high-performance liquid chromatography are as follows:

| | |
|---|---|
| Column: | YMC-packed column AM-312 (S-5 120A 0DS) |
| Mobile phase: | $CH_3CN$ : 0.01 M $(NH_4)_2CO_3$ag = 1800 : 1200 |
| Flow rate: | 1 ml/min. |
| Temperature: | 45°C |
| Detection: | UV (254 nm) |
| Retention time: | VLB 12 min. |
| | AVLB 42.1 min. |

Example 67

The procedure was carried out in the same manner as in Example 66 except that AVLB was replaced by 21.2 mg of AVLB sulfate and 2 N HCl was not added. At this time, the conversion rate of AVLB was 67%, the yield of VLB was 33.5%, and the yield of LEU was 13%.

Examples 68 to 71

The procedure was carried out in the same manner as in Example 66 except that the amount of $NaBH_4$ was varied. The results are shown in Table 19.

Table 19

| Example | $NaBH_4$ (mg/2 ml $H_2O$) | Conversion rate of AVLB (%) | Yield of VLB (%) | Yield of LEU (%) |
|---|---|---|---|---|
| 68 | 17.2 | 83.5 | 36 | 13 |
| 69 | 25.8 | 78.5 | 34 | 14 |
| 70 | 34.4 | 86 | 36.5 | 19 |
| 71 | 43.0 | 84 | 36.5 | 15.5 |

Examples 72 to 73

The procedure was carried out in the same manner as in Example 67 except that twice the amount of AVLB sulfate was employed and the amount of $NaBH_4$ was varied. The results are shown in Table 20.

Table 20

| Example | $NaBH_4$ (mg/2 ml $H_2O$) | Conversion rate of AVLB (%) | Yield of VLB (%) | Yield of LEU (%) |
|---|---|---|---|---|
| 72 | 17.2 | 56.5 | 27 | 10 |
| 73 | 34.4 | 69 | 34.5 | 15 |

Examples 74 to 75

The procedure was carried out in the same manner as in Example 66 except that the contact time was about 6 sec.; 38 mg of AVLB was employed; and the amount of $NaBH_4$ was varied. The results are shown in Table 21.

Table 21

| Example | $NaBH_4$ (mg/2 cc $H_2O$) | Conversion rate of AVLB (%) | Yield of VLB (%) | Yield of LEU (%) |
|---|---|---|---|---|
| 74 | 25.8 | 70.5 | 28.5 | 11 |
| 75 | 34.4 | 76.5 | 33 | 14 |

Example 76

The procedure was carried out in the same manner as in Example 66 except that 38 mg of AVLB was employed; the amount of $NaBH_4$ was 34.4 mg; and the bubbling gas into the AVLB solution was changed to pure oxygen. At this time, the conversion rate of AVLB was 72.5%, the yield of VLB was 27.5%, and the yield of LEU was 11%.

Example 77

The procedure was carried out in the same manner as in Example 67 except that the amount of $NaBH_4$ was 34.4 mg, and that α,α'-bipyridine equivalent to the trivalent iron was added in place of $NH_4Cl$. At this time, the conversion rate of AVLB was 65%, the yield of VLB was 36%, and the yield of LEU was 9%.

Example 78

The procedure was carried out in the same manner as in Example 70 except that $FeCl_36H_2O$, $(NH_4)_2C_2O_4$, $NH_4Cl$ and glycine in the AVLB sulfate solution in Example 5 was prepared separately as 2 cc of an aqueous solution, and then three types of the solutions, i.e. this aqueous solution, AVLB sulfate solution, and $NaBH_4$ solution were simultaneously and continuously introduced into the column. At this time, the conversion rate of AVLB was 80%, the yield of VLB was 31%, and the yield of LEU was 12%.

Example 79

In a 300 ml-reaction vessel placed 100 ml of $H_2O$, 19 mg of anhydrovinblastine, 30-fold molar excess $FeCl_36H_2O$, 5-molar excess $H^+$(2 N HCl), and equivalent molar α,α'-pyridyl, based on the anhydrovinblastine, 2-fold molar excess $(NH_4)_2C_2O_4$ based on the $Fe^{3+}$. The mixture was stirred over 30 min. under cooling in ice while air was bubbled. Following addition of 2 ml of an aqueous solution of $NaBH_4$ (0.227 M), the mixture was stirred for an additional 30 min. Addition of 10 ml of 25% ammonia water made the mixture basic. Then, the product was extracted with 100 ml of ethyl acetate, 3 times. The extracts were combined and evaporated to dryness under reduced pressure at 40°C or less. The product was analyzed by high-performance liquid chromatography. At this time, the conversion rate of anhydrovinblastine (AVLB) was 80%, the yield of vinblastine of (VLB) was 50% (selectively 63%), and the yield of leurosidine (LEU) was 13%.

(Analytical conditions)

| | |
|---|---|
| Column: | YMC-packed column AM-312 (S-5 120A 0DS) |
| Mobile phase: | $CH_3CN$ : 0.01 M $(NH_4)_2CO_3$ = 1800 : 1200 (V/V) |
| Flow rate: | 1 ml/min. |
| Column temperature: | 45°C |
| Detection: | UV (254 nm) |
| Retention time: | VLB 12.8 min. |

LEU 10.8 min.
AVLB 42 min.

Example 80

The procedure was carried out in the same manner as in Example 79 except that α,α'-bipyridyl in Example 79 was not added. At this time, the conversion rate of AVLB was 92%, the yield of VLB was 44% (selectivity 48%), and the yield of LEU was 19%.

Example 81

The procedure was carried out in the manner as in Example 79 except that α,α'-bipyridyl in Example 79 was replaced by 4,4'-dimenthy-2,2'-bipyridyl. At this time, the conversion rate of AVLB was 81%, the yield of VLB was 45% (selectivity 56%), and the yield of LEU was 17%.

Example 82

The procedure was carried out in the same manner as in Example 79 except that α,α'-bipyridyl was replaced by pyridine, and that the addition amount of pyridine was changed to 2.16 molar excess based on $Fe^{3+}$. At this time, the conversion rate of AVLB was 48%, the yield of VLB was 28% (selectivity 58%), and the yield of LEU was 4%.

Example 83

The procedure was carried out in the same manner as in Example 79 except that there was added 4-fold molar excess glycine based on $Fe^{3+}$. At this time, the conversion rate of AVLB was 75%, the yield of VLB was 50% (selectivity 67%), and the yield of LEU was 14%.

## Claims

1. A process for the preparation of a compound of general formula (II);

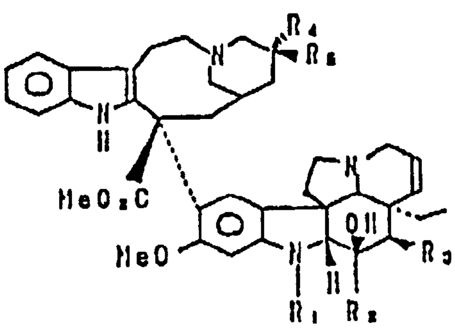

(II)

wherein $R_1$ represents hydrogen, a $C_1$-$C_4$ alkyl group or formyl group, $R_2$ represents a $C_1$-$C_4$ alkoxycarbonyl group or amide group, $R_3$ represents an acetoxy group or hydroxy group and $R_4$ represents-OH and $R_5$ represents ethyl or $R_4$ represents ethyl and $R_5$ represents -OH, which process comprises reacting a compound of general formula (I):

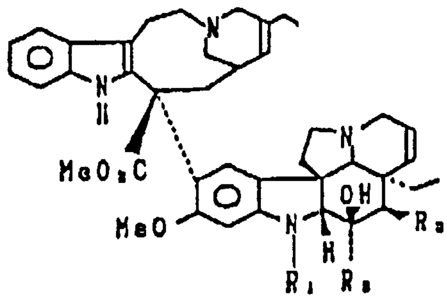

( I )

wherein $R_1$ to $R_3$ are as defined for formula (II) or a salt thereof, with oxygen in the presence of a trivalent iron source and a hydride source.

2. A process according to claim 1, wherein the reaction is carried out in the presence also of (i) an inorganic anion source which does not form an insoluble iron salt, and/or (ii) an axalic acid ion and/or a malonic acid ion source.

3. A process according to claim 2, wherein the reaction is carried out in the presence of (ii) and also pyridine or a pyridine derivative.

4. A process according to claim 2, wherein the reaction is carried out in the presence of (ii) and also a dicarboxylic acid of general formula (III);

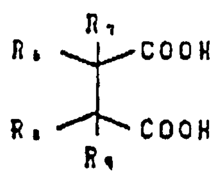

( III )

wherein $R_7$ and $R_9$ each represent hydrogen or a $C_1$-$C_4$ alkyl group, and $R_6$ and $R_6$ each represent hydrogen or a $C_1$-$C_4$ alkyl group or together form a double bond, or a salt thereof.

5. A process according to any one of the preceding claims wherein the reaction is carried out also in the presence of an amino acid.

6. A process according to any one of the preceding claims, wherein the compound of formula (II) is vinblastine (when the symbols in the formula are as follows: $R_1$=$CH_3$, $R_2$=$CO_2CH_3$, $R_3$=$OCOCH_3$, $R_4$=$C_2H_5$, and $R_5$=OH).

7. A process according to any one of claims 1 to 5, wherein the compound of formula (II) is leurosidine (when the symbols in the formula are as follows: $R_1$=$CH_3$, $R_2$=$CO_2CH_3$, $R_3$=$OCOCH_3$, $R_4$=OH, and $R_6$=$C_2H_5$).

8. A process according to any one of the preceding claims, wherein the compound of formula (I), a hydride source, oxygen and trivalent iron source are contained in two or more solutions, and then said two or more solutions are allowed to contact continuously with each other.

9. A process according to claim 8, wherein one or more components selected from an inorganic anion source, a pyridine derivative, a dicarboxylic acid of formula (III) or salt thereof as defined in claim 4, and an amino acid, together with an oxalic acid ion source and/or a malonic ion source, are included in one or two solutions among two or more solutions to be contacted.

**Patentansprüche**

1. Verfahren zur Herstellung einer Verbindung der allgemeinen Formel (II):

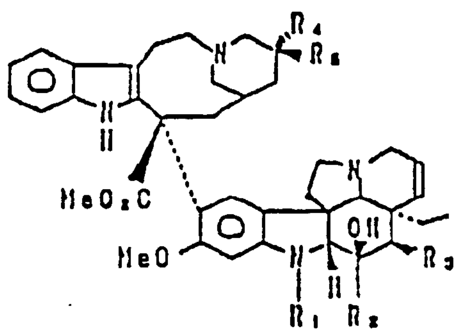

( II )

worin bedeuten:

$R_1$ Wasserstoff, eine $C_1$-$C_4$ Alkylgruppe oder eine Formylgruppe,
$R_2$ eine $C_1$-$C_4$ Alkoxycarbonylgruppe oder eine Amidgruppe,
$R_3$ eine Acetoxygruppe oder eine Hydroxygruppe, und
$R_4$ OH und $R_5$ Ethyl, oder
$R_4$ Ethyl und $R_5$ OH,

dadurch gekennzeichnet, dass eine Verbindung der allgemeinen Formel (I):

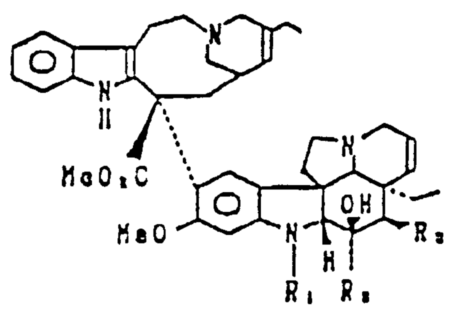

( I )

worin $R_1$ bis $R_3$ die in Formel (II) angegebene Bedeutung haben, oder ein Salz davon, mit Sauerstoff in Anwesenheit eines dreiwertigen Eisen-Ausgangsstoffes und eines Hydrid-Ausgangsstoffes zur Reaktion gebracht wird.

2. Verfahren gemäss Anspruch 1, dadurch gekennzeichnet, dass die Reaktion auch in Anwesenheit von (i) einem anorganischen Anion-Ausgangsstoff, welcher kein unlösliches Einsensalz bildet, und/oder (ii) einem Oxalsäure-Ion Ausgangsstoff und/oder einem Malonsäure-Ion Ausgangsstoff durchgeführt wird.

3. Verfahren gemäss Anspruch 2, dadurch gekennzeichnet, dass die Reaktion in Anwesenheit von (ii) und auch Pyridin oder einem Pyridinderivat durchgeführt wird.

4. Verfahren gemäss Anspruch 2, dadurch gekennzeichnet, dass die Reaktion in Anwesenheit von (ii) und auch einer Dicarbonsäure der allgemeinen Formel (III):

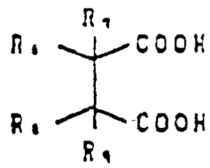

(III)

durchgeführt wird, worin bedeuten, jedes von $R_7$ und $R_9$ Wasserstoff oder eine $C_1$-$C_4$ Alkylgruppe, und jedes von $R_6$ und $R_8$ Wasserstoff oder eine $C_1$-$C_4$ Alkylgruppe oder $R_6$ und $R_8$ bilden zusammen eine Doppelbindung, oder ein Salz davon.

5. Verfahren gemäss einem der vorhergehenden Ansprüche, dadurch gekennzeichnet, dass die Reaktion auch in Anwesenheit einer Aminosäure durchgeführt wird.

6. Verfahren gemäss einem der vorhergehenden Ansprüche, dadurch gekennzeichnet, dass die Verbindung der Formel (II) Vinblastin ist (wenn die Symbole in der Formel sind: $R_1 = CH_3$, $R_2 = CO_2CH_3$, $R_3 = OCOCH_3$, $R_4 = C_2H_5$ und $R_6 = OH$).

7. Verfahren gemäss einem der Ansprüche 1 bis 5, dadurch gekennzeichnet, dass die Verbindung der Formel (II) Leurosidin ist (wenn die Symbole in der Formel sind: $R_1 = CH_3$, $R_2 = CO_2CH_3$, $R_3 = OCOCH_3$, $R_4 = OH$ und $R_6 = C_2H_5$).

8. Verfahren gemäss einem der vorhergehenden Ansprüche, dadurch gekennzeichnet, dass die Verbindung der Formel (I), ein Hydrid-Ausgangsstoff, Sauerstoff und ein dreiwertiger Eisen-Ausgangsstoff in zwei- oder mehr Lösungen enthalten sind, und dass dann diese zwei oder mehr Lösungen kontinuierlich miteinander in Berührung gebracht werden.

9. Verfahren gemäss Anspruch 8, dadurch gekennzeichnet, dass ein oder mehrere Komponenten, ausgewählt aus einem anorganischen Anion-Ausgangsstoff, einem Pyridinderivat, einer Dicarbonsäure der Formel (III) oder einem Salz davon, wie in Anspruch 4 definiert, und eine Aminosäure, zusammen mit einem Oxalsäure-Ion-Ausgangsstoff und/oder einem Malonsäure-Ion-Ausgangsstoff, in ein oder zwei Lösungen unter zwei oder mehr Lösungen die in Berührung gebracht werden, enthalten sind.

**Revendications**

1. Procédé de préparation d'un composé de formule générale (II) :

(II)

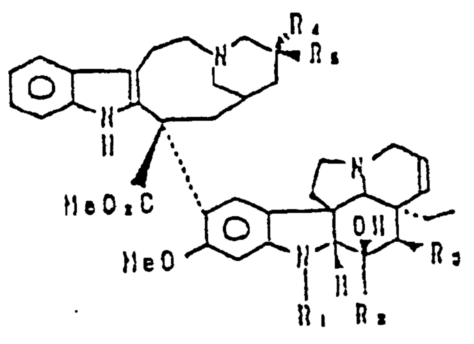

dans laquelle $R_1$ représente un atome d'hydrogène ou un groupe alkyle en $C_1$-$C_4$ ou un groupe formyle, $R_2$ représente un groupe (alkoxy en $C_1$-$C_4$)-carbonyle ou un groupe amido, $R_3$ représente un groupe acétoxy ou un groupe hydroxy, et $R_4$ représente un groupe hydroxy et $R_5$ représente un groupe éthyle ou bien $R_4$ représente un groupe éthyle et $R_5$ représente un groupe hydroxy,
lequel procédé comporte la réaction d'un composé de formule générale (I)

(I)

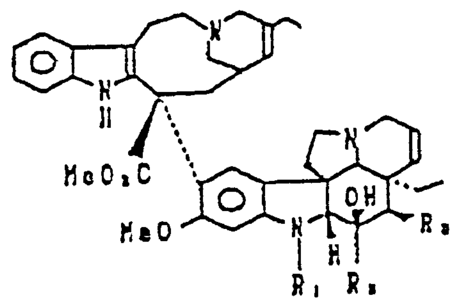

dans laquelle $R_1$ à $R_3$ sont définis comme pour la formule (II), ou d'un sel d'un tel composé, avec de l'oxygène, en présence d'une source de fer trivalent et d'une source d'hydrure.

2. Procédé conforme à la revendication 1, dans lequel la réaction est effectuée en présence aussi de (i) une source d'un anion inorganique qui ne forme pas un sel insoluble de fer, et/ou (ii) une source d'ion oxalate et/ou d'ion malonate.

3. Procédé conforme à la revendication 2, dans lequel la réaction est effectuée en présence de la substance (ii) et aussi de pyridine ou d'un dérivé de pyridine.

4. Procédé conforme à la revendication 2, dans lequel la réaction est effectuée en présence de la substance (ii) et aussi d'un acide dicarboxylique de formule générale (III) :

(III)

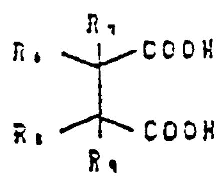

dans laquelle $R_7$ et $R_9$ représentent chacun un atome d'hydrogène ou un groupe alkyle en $C_1$-$C_4$ et $R_6$ et $R_8$ représentent chacun un atome d'hydrogène ou un groupe alkyle en $C_1$-$C_4$ ou forment ensemble une double liaison, ou d'un sel d'un tel acide.

5. Procédé conforme à l'une quelconque des revendications précédentes, dans lequel la réaction est effectuée en présence aussi d'un acide aminé.

6. Procédé conforme à l'une quelconque des revendications précédentes, dans lequel le composé de formule (II) est la vinblastine (quand, dans la formule, les symboles ont les significations suivantes : $R_1 = CH_3$ ; $R_2 = CO_2CH_3$ ; $R_3 = OCOCH_3$ ; $R_4 = C_2H_5$; et $R_5$ = OH).

7. Procédé conforme à l'une quelconque des revendications 1 à 5, dans lequel le composé de formule (II) est la leurosidine (quand, dans la formule, les symboles ont les significations suivantes : $R_1 = CH_3$ ; $R_2 = CO_2CH_3$; $R_3 = OCOCH_3$; $R_4$ = OH; et $R_6 = C_2H_5$).

8. Procédé conforme à l'une quelconque des revendications précédentes, dans lequel le composé de formule (I), la source d'hydrure, l'oxygène et la source de fer trivalent sont contenus dans deux solutions ou plus, et l'on fait alors entrer ces deux solutions ou plus en contact les unes avec les autres, en continu.

9. Procédé conforme à la revendication 8, dans lequel un ou plusieurs composants choisis parmi une source d'anion inorganique, un dérivé de pyridine, un acide dicarboxylique de formule (III) ou un sel d'un tel acide tel que défini dans la revendication 4, et un acide aminé, conjointement avec une source d'ion oxalate et/ou une source d'ion malonate, sont contenus dans une ou deux solutions parmi les deux solutions ou plus à faire entrer en contact.